Europäisches Patentamt

European Patent Office

Office européen des brevets

(11) Numéro de publication : **0 461 009 A1**

# (12) DEMANDE DE BREVET EUROPEEN

(21) Numéro de dépôt : **91401414.7**

(51) Int. Cl.⁵ : **A61B 5/0436**

(22) Date de dépôt : **30.05.91**

(30) Priorité : 30.05.90 FR 9006690

(43) Date de publication de la demande :
**11.12.91 Bulletin 91/50**

(84) Etats contractants désignés :
**AT BE CH DE DK ES FR GB GR IT LI LU NL SE**

(71) Demandeur : **Société Anonyme MEDIAN
37, rue des Petits Ruisseaux, Z.A. Des Godets
Verrières Le Buisson (Essonne) (FR)**

(72) Inventeur : **l'Henoret, Louis Hervé Yvon
115 Avenue François Molé
Antony, Hauts de Seine (FR)**

(74) Mandataire : **Rataboul, Michel Charles
CMR INTERNATIONAL 69, rue de Richelieu
F-75002 Paris (FR)**

(54) **Procédé pour l'enregistrement et la lecture de signaux, notamment d'électrocardiogrammes et dispositif pour la mise en oeuvre de ce procédé.**

(57) Le procédé est destiné à l'enregistrement et à la lecture de signaux, notamment d'électrocardio-grammes.

Il est caractérisé en ce que l'on enregistre en continu la totalité des signaux émis, dans au moins une mémoire solide, que l'on transfère périodiquement lesdits signaux sur un support d'enregistrement après leur avoir fait subir une modulation, que l'on enregistre sur ledit support d'enregistrement une fréquence de référence, ladite fréquence servant à la démodulation des signaux, que l'on traite les signaux en temps réel lors de l'enregistrement, que l'on stocke les informations obtenues dans une partie de la mémoire solide qui n'est pas affectée lors des transferts périodiques, afin de pouvoir lire ces informations sans faire appel au support d'enregistrement comportant un tracé complet des signaux captés.

EP 0 461 009 A1

FIG.3

La présente invention concerne un dispositif d'enregistrement et de lecture de signaux notamment d'électrocardiogrammes, ou "ECG".

On connaît des dispositifs pour la surveillance des rythmes cardiaques "en ambulatoire", c'est-à-dire dans des conditions qui permettent au patient de marcher, de se déplacer et, cela, par opposition aux installations qui exigent l'immobilité du patient, généralement couché dans un lit.

Ces dispositifs classiques, encore appelés "holters", sont de deux types principaux : le holter à cassette à bande magnétique, et le holter à mémoire solide.

Les holters à cassette à bande magnétique ont l'avantage d'avoir une certaine souplesse d'emploi lors de la lecture, car il peuvent faire avancer la bande magnétique à deux ou trois vitesse de défilement différentes. Mais la mécanique d'entraînement s'use rapidement car, lors de l'enregistrement, la bande doit être entraînée à vitesse très lente. En effet, pour réaliser un enregistrement pendant 24 heures avec une bande de 132 mètres de longueur, il faut faire défiler la bande à la vitesse de :

$$\frac{13.200}{24 \, . \, 60 \, . \, 60} = 0,15 \; cm/sec$$

La bande passante du dispositif est inférieure au spectre du signal ECG qui va de 0,5 Hz à 1 KHz, par suite de la durée très brève des cinq oscillations présentées par ce signal : P (systole auriculaire) QRST (systole ventriculaire).

Les holters à mémoires solide, quant à eux, ont l'avantage d'être fiables, mais leur coût est élevé, compte tenu du volume des informations à stocker sous forme numérique. Par exemple, pour stocker un signal ECG pendant 24 heures, la capacité minimale de la mémoire doit être de :

150 . 60 . 60 . 24 octets = 12,96 M octets par voie,

si l'on prend une fréquence minimale du spectre de l'électrocardiogramme (0,05 à 50 Hz. En outre, les dispositifs à mémoire solide ne permettent pas une lecture accélérée et variable de façon continue, comme cela est le cas des enregistreurs à bande magnétique.

Afin de réduire la taille des mémoires solides nécessaires, des holters comprennent un circuit de compression des données en opérant par reconnaissance de formes. Or, la difficulté dans la reconnaissance de formes est dans la détection, car l'ordinateur rejette précisément les signaux les plus intéressants pour reconnaître la pathologie, car ils ne sont évidemment pas conformes aux modèles de comparaison. En effet, quand un patient a une crise, il souffre et bouge, ce qui génère des parasites qui perturbent les signaux à un moment crucial.

On connaît des ordinateurs qui enregistrent des signaux dans une mémoire tampon et les vident périodiquement sur une bande magnétique à une vitesse plus rapide que la vitesse d'enregistrement. Ces dispositifs ne s'appliquent pas à la surveillance du rythme cardiaque, car la lecture des bandes ne peut actuellement s'effectuer qu'à vitesse fixe ou par paliers.

Pour fixer l'état de la technique on peut citer le brevet EP-A-003.797 qui décrit un appareil de traitement d'une information, sous la forme de signaux mémorisés sur bande magnétique, en vue de mesurer dans le temps des complexes de signaux essentiellement périodiques, tels que par exemple des électrocardiogrammes.

Cet appareil comporte au moins un compteur associé à au moins une mémoire qui sont destinés à compter et/ou à mémoriser des impulsions à cadences normalisées, qui peuvent être mis en fonctionnement sous l'action d'un circuit de commande détectant un événement significatif en forme de signal et qui peuvent être remis à zéro, soit à la fin de l'événement significatif par le déclenchement d'un signal approprié, soit par l'apparition d'un second événement significatif.

Or, Un tel appareil ne fonctionne pas en continu, ce qui peut être très préjudiciable au patient qui le porte, car il ne détecte que des événements correspondant à des signaux enregistrés dont la valeur correspond à seuil déterminé, sans prendre en considération les signaux qui apparaissent préalablemnt à ceux de l'événement car leur valeur est plus faible.

Une telle détection peut entraîner de sérieuses conséquences car dès la détection des signaux révélateurs de l'événement, le patient peut déjà se trouver dans un état physilogique grave, ce qui diminue évidemment l'efficacité d'une intervention médicale. En outre, la non connaissance de l'historique de l'événement rend plus délicate l'appréciation par le médecin de l'état pathologique dans lequel se trouve le patient et rend plus difficile son diagnostic.

Par ailleurs, cet appareil ne permet pas d'assurer un traitement des signaux à l'enregistrement, ce qui est une source d'erreurs en raison des variations de la vitesse de défilement de la bande lors de l'enregistrement.

Il en résulte que ces erreurs nuisent considérablement à l'efficacité de l'appareil et font courir de graves dangers au patient puisque les signaux enregistrés manquent de fiabilité.

On connait également le brevet GB-A-2.034.046 qui décrit un dispositif électronique complexe qui n'assure un enregistrement des signaux électriques qu'après l'apparition d'un accident pathologique et, par conséquent, qui ne fonctionne pas en continu.

Un tel dispositif ne permet pas d'assurer une sécurité au patient car il faut pour qu'il se mette en fonctionnement faut que le signal détecté dépasse un seuil prédéterminé, ce qui se produit précisément quand le patient est déjà dans un état pathologique assez grave.

Le brevet US-A-4.696.306 décrit un dispositif comprenant une horloge qui commande et contrôle la vitesse de défilement d'une bande magnétique sur laquelle sont enregistrées les informations sous forme de signaux. Il fonctionne en utilisant une boucle de régulation et une modulation en amplitude sans intervenir sur la vitesse de défilement de la bande et donc sans remédier aux erreurs résultant des variations de celle-ci.

Le brevet US-A-4.883.063 décrit un dispositif qui detecte un signal de référence en amplitude et qui effectue une modulation en amplitude de ce signal à partir d'un certain seuil pour obtenir un gain en amplitude quasi constant.

Ce dispositif présente l'inconvénient d'être peu fiable car une variation en amplitude du signal entraîne une variation de la vitesse de défilement de la bande magnétique sur laquelle sont enregistrées les informations, ce qui entraîne une erreur d'enregistrement des signaux.

Enfin, on peut citer le document US-A-3.650.263 décrivant un dispositif électronique complexe qui fonctionne uniquement quand le signal enregistré dépasse un seuil prédéterminé.

Le but de la présente invention est de fournir un dispositif d'enregistrement et de lecture d'ECG, présentant des moyens d'enregistrement fiables, fidèles, peu coûteux à l'achat et à l'entretien, et des moyens de lecture offrant une grande souplesse d'utilisation.

A cette fin, l'invention a pour objet un procédé pour l'enregistrement et la lecture de signaux, notamment d'électrocardiogrammes, caractérisé en ce que l'on enregistre en continu la totalité des signaux émis, dans au moins une mémoire solide, que l' on transfère périodiquement lesdits signaux sur un support d'enregistrement après leur avoir fait subir une modulation, que l'on enregistre sur ledit support d'enregistrement une fréquence de référence, ladite fréquence servant à la démodulation des signaux, que l'on traite les signaux en temps réel lors de l'enregistrement, que l'on stocke les informations obtenues dans une partie de la mémoire solide qui n'est pas affectée lors des transferts périodiques, afin de pouvoir lire ces informations sans faire appel au support d'enregistrement comportant un tracé complet des signaux captés.

Selon d'autres caractéristiques de ce procédé :

— le support d'enregistrement est une bande magnétique et lors de la lecture, on compte la période de la fréquence de référence et la période de la fréquence des signaux en utilisant comme fréquence d'horloge une fréquence multiple de la fréquence de référence et on fait la différence de l'inverse des périodes obtenues de façon à restituer les signaux enregistrés en s'affranchissant de la vitesse et des variations de vitesse de la bande magnétique, aussi bien à l'enregistrement qu'à la lecture;

— on transfère périodiquement des signaux de la mémoire solide au support d'enregistrement à une vitesse supérieure à la vitesse d'écriture dans la mémoire solide;

— lors de l'enregistrement, on choisit comme vitesse de déroulement de la bande magnétique une vitesse standard telle que neuf centimètres par seconde;

— on enregistre les signaux multiplexés en modulation de fréquence sur un support d'enregistrement, puis on les démultiplexe afin de les reconnaître;

— on amplifie les signaux et on compense le gain qui en résulte ainsi que des variations de paramètres affectant l'enregistrement des signaux tels que la vitesse d'enregistrement;

— on synchronise les signaux à enregistrer et on les repère par rapport à un signal de référence.

L'invention a également pour objet un dispositif pour l'enregistrement et la lecture de signaux, notamment d'électrocardiogrammes, du type comprenant un support d'enregistrement, des moyens d'entraînement du support d'enregistrement, une tête d'écriture et une tête de lecture reliées respectivement à un bloc électronique d'écriture et à un bloc électronique de lecture, caractérisé en ce qu'il présente une liaison entre les blocs électroniques et les têtes, liaison destinée à un signal de référence de vitesse.

Selon d'autres caractéristiques de ce dispositif :

— il comprend une cassette à bande magnétique, des moyens d'entraînement de la bande magnétique, une tête magnétique d'enregistrement et une tête magnétique de lecture reliées respectivement à un bloc électronique d'enregistrement et à un bloc électronique de lecture et est caractérisé en ce qu'il présente une liaison entre les blocs électroniques et les têtes magnétiques, liaison déstinée à un signal de référence de vitesse;

— il est du type comprenant des moyens d'enregistrement pouvant recevoir une cassette et reliés à des capteurs, et est caractérisé en ce qu'il possède une liaison reliant le bloc électronique d'enregistrement et la tête magnétique d'enregistrement, liaison déstinée à un signal de référence de vitesse;

– il est du type comprenant des moyens de lecture pouvant recevoir une cassette et reliés à un ordinateur et/ou à une imprimante et est caractérisé en ce qu'il possède une liaison reliant le bloc électronique de lecture et la tête magnétique de lecture, liaison destinée un signal de référence de vitesse;

– le bloc électronique d'enregistrement comprend un oscillateur à quartz pour générer une fréquence servant de signal de référence de vitesse;

– le dispositif comprend un oscillateur contrôlé en tension dont la fréquence centrale est égale à la fréquence de l'oscillateur à quartz;

– le bloc électronique de lecture comprend un circuit multiplicateur destiné à multiplier le signal de référence de vitesse par une constante;

– le circuit multiplicateur est un circuit à verrouillage de phase;

– le bloc électronique de lecture comprend des circuits compteurs devant être associés à des moyens d' horloge de comptage, ces moyens étant constitués par la fréquence multiple de la fréquence du signal de référence, délivrée par le circuit multiplicateur;

– le bloc électronique d'enregistrement présente une mémoire solide et des décodeurs d'adresse dont la fréquence de travail est égale ou sous-multiple de la fréquence de référence lors de l'écriture et est égale ou multiple de la fréquence de référence lors de la lecture;

– le bloc électronique d'enregistrement comporte au moins une voie destinée à recevoir un signal sous forme analogique, une voie destinée à recevoir un signal de référence calibré et une voie de mise à zéro;

– le bloc électronique d'enregistrement comporte un multiplexeur associé à des moyens de modulation en fréquence et à un organe de décodage d'adresses;

– le bloc électronique de lecture comporte un démultiplexeur associé à un échantillonneur;

– les moyens d'entraînement comprennent un moteur qui est relié soit directement, soit au moyen d'une simple courroie, à une poulie solidaire d'un embout d'entraînement devant être en prise avec une bobine d'une cassette;

– les moyens d'entraînement comprennent deux moteurs qui sont coaxiaux à deux bobines d'une cassette;

– les moyens d'entraînement sont associés à un ensemble optoélectronique de contrôle et de régulation de la vitesse de la bande magnétique d'une cassette.

L'invention sera mieux comprise par la description détaillée ci-après faite en référence au dessin annexé. Bien entendu, la description et le dessin ne sont donnés qu'à titre d'exemple indicatif et non limitatif.

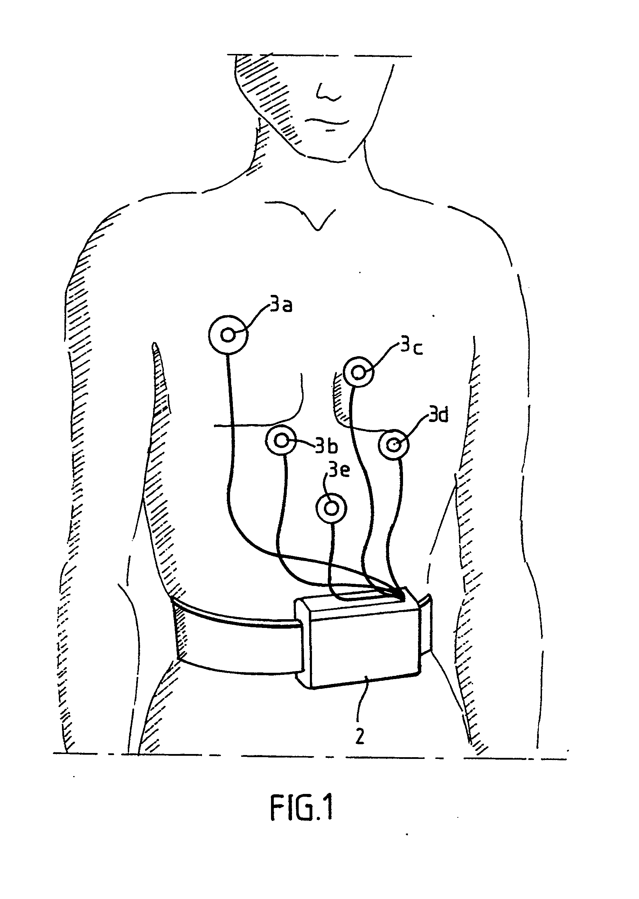

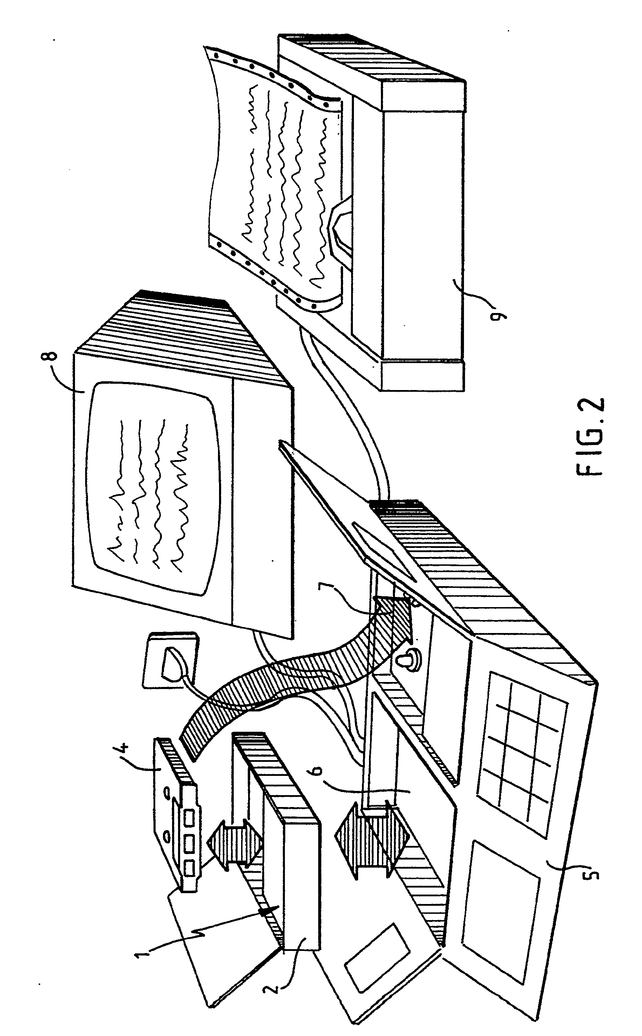

La Figure 1 illustre schématiquement des moyens d'enregistrement conformes à l'invention, portés par un patient.

La Figure 2 illustre schématiquement des moyens de lecture conformes à l'invention, dont les compartiments pour les moyens d'enregistrement et pour une cassette amovible sont ouverts.

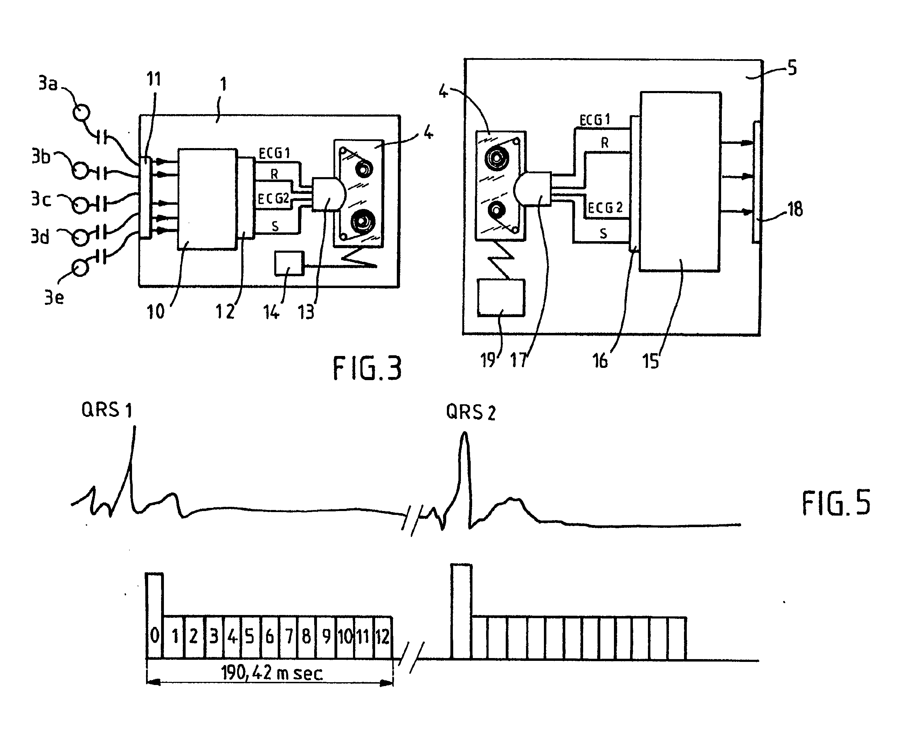

La Figure 3 est un schéma général d'un dispositif conforme à l'invention, respectivement pour les moyens d'enregistrement et pour les moyens de lecture.

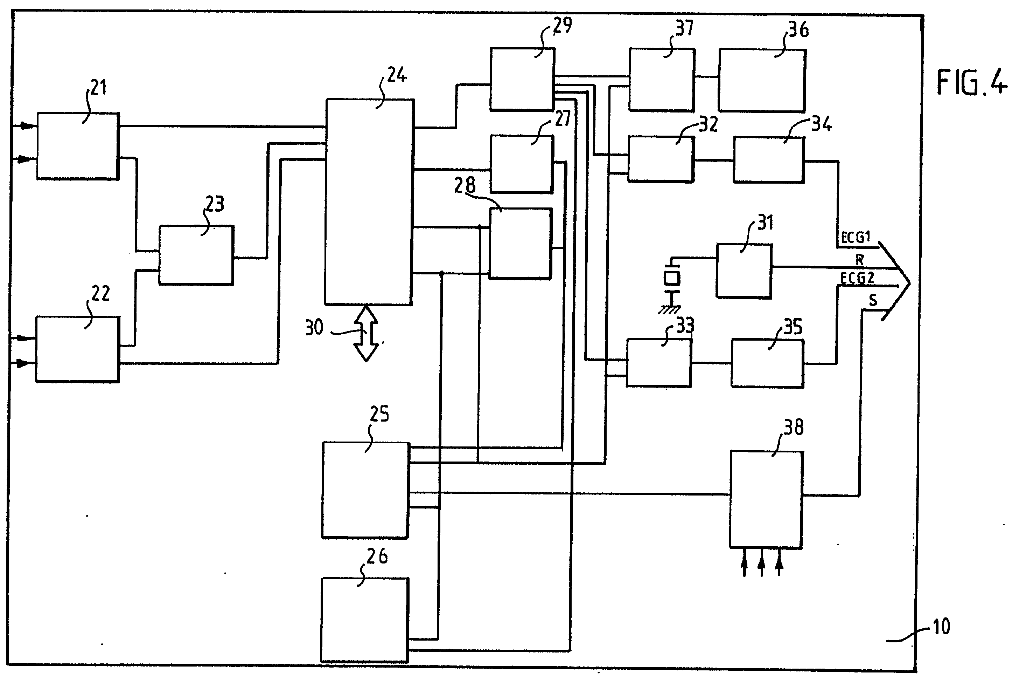

La Figure 4 est un schéma du bloc électronique d'enregistrement conforme à l'invention.

La Figure 5 est une illustration schématique de la partie d'un électrocardiogramme connue par l'appellation "QRS".

La Figure 6 est un schéma du bloc électronique de lecture conforme à l'invention.

La Figure 7 est un schéma d'un multiplicateur de fréquence conforme à l'invention.

La Figure 8 est une vue schématique en plan des moyens d'entraînement d'une cassette à bande magnétique lors d'un enregistrement.

La Figure 9 est une vue schématique en plan des moyens d'entraînement d'une cassette à bande magnétique lors d'une lecture.

La figure 10 est un schéma général d'un dispositif conforme à une variante de réalisation de l'invention, respectivement pour les moyens d'enregistrement et les moyens de lecture.

En se reportant au dessin, on voit un dispositif d'enregistrement et de lecture conforme à l'invention et qui comprend :

– des moyens d'enregistrement 1 placés dans un boîtier 2 porté par un patient et reliés à des capteurs 3a; 3b, 3c, 3d, 3e, et contenant une cassette 4 à bande magnétique, de type standard et montée de façon amovible;

– des moyens de lecture 5, présentant un logement 6 pour recevoir les moyens d'enregistrement 1 et un logement 7 pour recevoir la cassette 4, le tout étant relié à un calculateur 8 et à une imprimante 9 du commerce.

Les moyens d'enregistrement 1 comprennent un bloc électronique 10, dit "bloc électronique d'enregistrement", dont l'entrée 11 est reliée aux capteur 3a, 3b, 3c, 3d, 3e, et dont la sortie 12 est reliée à une tête d'écriture 13 et des moyens d'entraînement 14 de la bande magnétique de la cassette 4.

Les moyens de lecture 5 comprennent un bloc électronique 15, dit "bloc électronique de lecture", dont l'entrée 16 est reliée à une tête de lecture 17, et dont la sortie 18 est reliée au calculateur 8 et à l'imprimante 9, et des moyens d'entraînement 19 de la bande magnétique de la cassette 4.

Entre le bloc électronique d'enregistrement 10 et la tête d'enregistrement 13 d'une part, et entre la tête de lecture 17 et le bloc électronique de lecture 15 d'autre part, on trouve quatre conducteurs repérés de la façon suivante :
- un conducteur pour une première voie d'électrocardiogramme repéré "ECG 1";
- un conducteur pour une seconde voie d'électrocardiogramme repéré "ECG 2";
- un conducteur pour un signal de synchronisation et un mot d'état QRS repéré "S";
- un conducteur pour un signal de référence de vitesse repéré "R".

La cassette à bande magnétique 4 est une cassette du commerce dont le ruban a une longueur de 132 mètres et une largeur permettant d'enregistrer quatre pistes, comprenant deux bobines mobiles autour de leurs axes.

Le bloc électronique d'enregistrement 10 comprend :
- des circuits 21, 22 de prétraitement du rythme des ECG et de détection du rythme cardiaque, ainsi qu'un circuit logique 23 de corrélation des voies;
- un microprocesseur 24 relié à des mémoires solides 25, à un horodateur 26, à des décodeurs d'adresse 27, 28, 29 et à un circuit 30 à la norme RS 232 bien connue de l'homme de métier;
- un oscillateur à quartz 31 délivrant une fréquence de référence égale à 8.192 Hz relié au conducteur R;
- des circuits convertisseurs numériques alanogiques 32, 33 suivis de circuits de modulation 34, 35 reliés aux conducteurs ECG 1 et ECG 2;
- un circuit 38 de modulation du signal de synchronisation et du mot d'état QRS relié au conducteur S;
- un écran à cristaux liquides 36, relié à des circuits de commandes 37 ayant pour but de :
  . afficher l'heure et la minute réelles pendant la phase où l'analyseur enregistreur est en fonction,
  . informer le médecin sur le bon déroulement du départ de l'enregistrement (autotest),
  . afficher en permanence la fréquence cardiaque moyenne, ainsi que les deux "tops" de détection de QRS,
  . indiquer par un marqueur ou un chiffre les défauts éventuels,
  . effectuer une vérification de bon fonctionnement, après la mise en place de la cassette 4.

Les signaux captés par les électrodes 3a, 3b, 3c, 3d, 3e sont traités par les circuits de prétraitement 21, 22. Après amplification, les signaux ECG sont convertis en signaux numériques par des circuits de type connu (non représentés) et transmis au microprocesseur 24. La fréquence d'échantillonage est de 8.192/60 = 136,53 Hz sur chaque voie, ce qui correspond à une période d'échantillonage de 7,32 millisecondes, et les deux voies sont échantillonnées alternativement, ce qui fait qu'il y a un point de mesure toutes les 3,66 millisecondes.

Par ailleurs, les circuits de prétraitement détectent le rythme cardiaque et les défauts sur les deux voies. Les signaux obtenus sont fournis au circuit logique de corrélation 23 qui les traite et les transmet au microprocesseur 24. Le microprocesseur 24 range les signaux des deux voies ECG 1 et ECG 2 dans un bloc de la mémoire solide 25, dit bloc principal. A chaque période d'échantillonnage correspondent huit positions mémoires pour chaque voie ECG 1 et ECG 2, sept positions servent à coder l'amplitude de l'ECG, la huitième position sert, sur une voie, à coder une impulsion de synchronisation et sur une autre voie à coder un mot d'état QRS fournissant les résultats de l'analyse temps réel des signaux.

Si les informations sont enregistrées pendant une durée de 16 minutes par exemple, le microprocesseur met en action les moyens d'entraînement 14 de la cassette 4, et lit les mémoires en 16 secondes.

Cet exemple n'est évidemment pas limitatif car le temps de stockage en mémoire numérique dépend uniquement de la capacité des mémoires. Il s'agit ici d'un bon compromis coût/temps de stockage.

Il existe donc un rapport de 60 entre la vitesse de lecture et la vitesse d'écriture dans la mémoire solide 25. Il est alors possible d'enregistrer sur la bande magnétique en 24 minutes un ECG d'une durée de 24 heures en prenant une bande de 132 mètres de longueur et en choisissant une vitesse linéaire sensiblement égale à 9 centimètres par seconde, c'est-à-dire 60 fois plus rapide que pour un enregistrement en l'absence de mémoire solide. On remarquera que 9 cm/s est une vitesse standard pour les enregistreurs magnétiques du commerce. On remarquera également que par rapport à un enregistreur classique, on réalise une importante économie d'énergie, l'enregistreur suivant l'invention ne fonctionnant que 24 minutes au lieu de 24 heures.

On ménage un blanc de 0,5 seconde pendant la période de démarrage de la cassette 4 et, pendant le transfert d'un bloc (16 s + 0,5 s), on continue le traitement pour ne pas perdre d'information. On range les informations dans un bloc de la mémoire solide 25, dit "bloc auxiliaire". Deux blocs auxiliaires sont prévus, de sorte que l'un se remplit pendant que l'autre se vide.

Une partie de la mémoire solide 25 accueille des programmes de fonctionnement du dispositif qui peuvent être téléchargés. La capacité de la mémoire 25 varie de 1 à 4 Mégaoctets suivant les réalisations. Cette capacité est nettement inférieure à la capacité de la mémoire des holters présentant une seule mémoire solide.

Il y a par conséquent trois mémoires :

– une mémoire solide pour les programmes, qui est téléchargeable,

– une mémoire solide pour le stockage des résultats des traitements, en temps réel,

– une mémoire de masse (ici une cassette à bande magnétique) pour l'enregistrement des traces des ECG.

Les deux premières mémoires solides peuvent avantageusement être deux parties d'une même mémoire solide.

Les signaux lus dans la mémoire solide 25 sont des signaux numériques. Avant enregistrement, on code ces signaux en modulation de fréquence pour les rendre compatibles avec la bande passante utile de l'ensemble d'enregistrement sur bande magnétique.

Le codage en fréquence des signaux ECG nécessite une conversion numérique/analogique préalable effectuée par les circuits 32, 33 et il est réalisé par les moyens de modulation 34, 35 qui présentent un oscillateur commandé en tension, c'est-à-dire un oscillateur dont la fréquence est de la forme :

$$f = fo + K Ve$$

où :

Ve est la tension de commande,

f est la fréquence centrale de l'oscillateur obtenue lorsque Ve = 0,

fo est une fréquence prise égale à la fréquence de l'oscillateur à quartz 31,

K est une constante de l'oscillateur s'exprimant en hertz/volt.

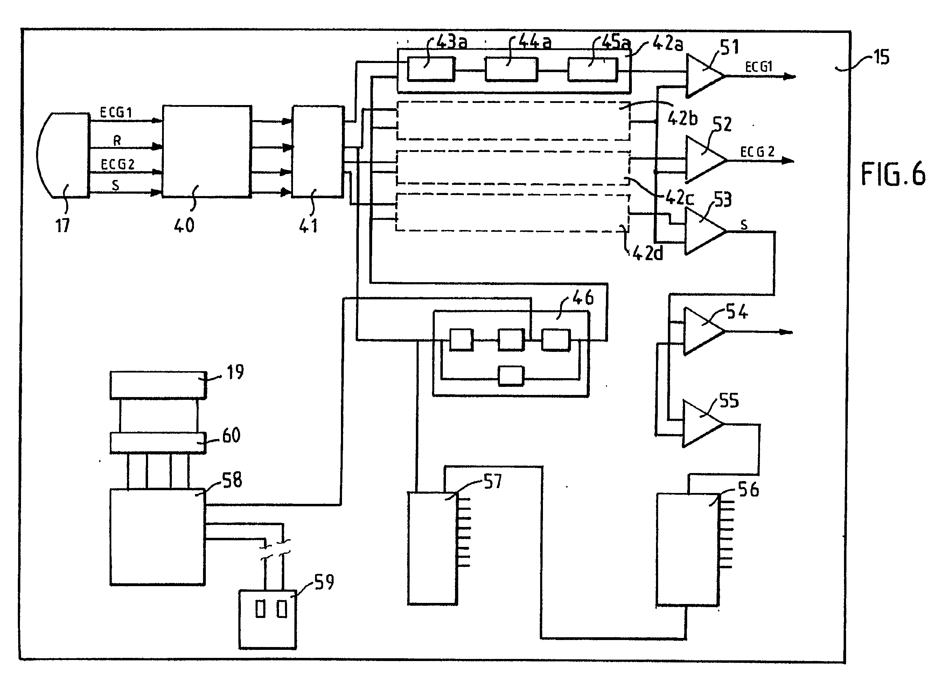

On voit sur la figure 5 un signal ECG et un train de treize impulsions dont chacune a une durée de 14,64 millisecondes, soit deux fois la période d'échantillonnage d'une voie ECG :

$$14,64 = \frac{60}{8192} \cdot 2$$

dont la durée totale est de :

14,64 . 13 = 190,32 millisecondes

chiffre inférieur à 200 millisecondes, durée minimale d'une pulsation cardiaque, correspondant à un rythme cardiaque de 300 pulsations par minute que l'on peut trouver chez le nourrisson.

Ce train d'impulsions est synchrone du rythme cardiaque. La première impulsion est une impulsion de synchronisation et les douze impulsions suivantes constituent le mot d'état QRS dont le code sera donné plus loin.

Afin que ce mot d'état arrive avant le QRS qu'il caractérise, les signaux cardiaques de l'ECG sont retardés de 32 périodes d'échantillonnage :

$$\frac{60}{8192} \cdot 32 = 234,47 \text{ millisecondes}$$

La composition du QRS est donc obtenue en temps réel :

234,37 msec - 190,42 msec = 43,95 msec avant le QRS.

Le codage des impulsions sur la voie S est le suivant :

| | | |
|---|---|---|
| niveau 0 | 136,52 . 60 = | 8192 Hz |
| niveau 1 | 200 . 60 = | 12000 Hz |
| niveau QRS | 256 . 60 = | 15360 Hz |

Le détail du mot d'état est le suivant :

bits 1, 2, 3, 4 : Morphologie du QRS (16 combinaisons)

5 : Défaut voie 1 ou artéfacts ++

6 : Défaut voie 2 ou artéfacts ++

7 : QRS précoce

7

8            : Période pathologique (marqueur S-T ou autres)

9            : Sortie enregistrée par l'analyseur

10           : Présence impulsion stimulation

11           : Pas de réponse stimulateur

12           : A définir

Comme on peut le voir à la figure 6, le bloc électronique de lecture 5 comprend un circuit préamplificateur 40 qui amplifie les signaux provenant des quatre voies de la tête de lecture 14, puis les transmet à un multiplicateur 41 qui les multiplie par 2, et les adresse à une première entrée de quatre circuits de comptage 42a, 42b, 42c, 42d, comprenant chacun un circuit de comptage périodique 43, un registre 44 et un convertisseur numérique/analogique multiplicateur 45. Ces quatre circuits de comptage présentent une deuxième entrée à laquelle est appliquée une fréquence provenant d'un multiplicateur 46 qui multiplie par 128 la fréquence de référence lue après qu'elle ait déjà été multipliée par 2 par le circuit 41.

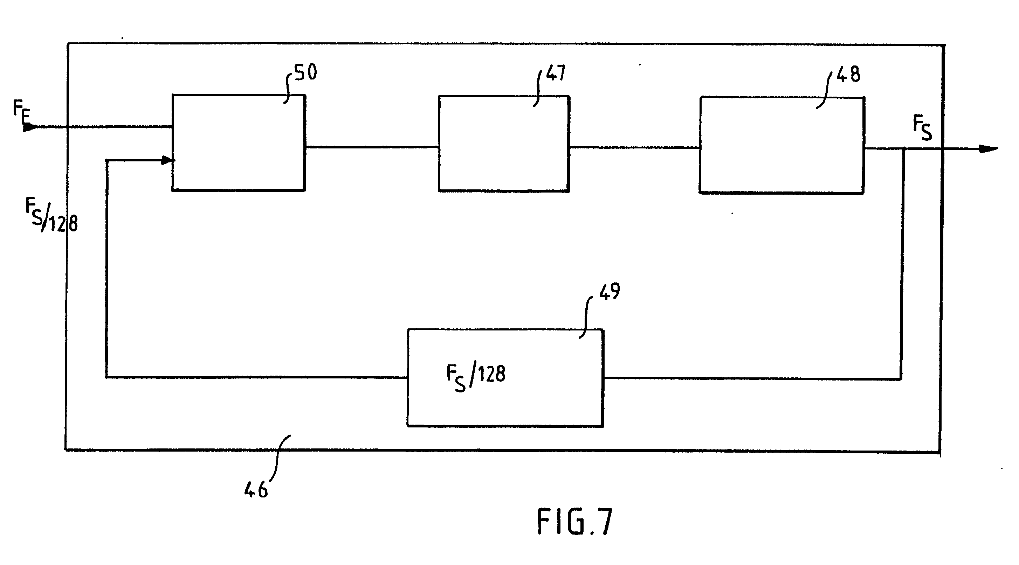

On voit sur la figure 7, que le circuit multiplicateur 46 est un circuit à contre-réaction comprenant dans la voie directe un filtre passe-bas 47 et un oscillateur contrôlé en tension 48 et, dans la boucle de retour, un circuit diviseur 49 qui divise par 128. Un comparateur de phase 50 compare la phase du signal d'entrée Fs divisée par 128. Le système trouve son équilibre lorsque :

$$Fe = \frac{Fs}{128}$$

c'est-à-dire lorsque :

$$Fs = 128 \, Fe$$

Les sorties des compteurs 42 sont reliées à trois amplificateurs opérationnels 51, 52, 53 qui reçoivent également la sortie du compteur 42b.

Les amplificateurs opérationnels 51, 52, 53 font la différence des signaux reçus et délivrent respectivement les signaux ECG 1, ECG 2 et S. L'amplificateur opérationnel 53 délivrant le signal S est relié en parallèle à deux amplificateurs opérationnels 54, 55 recevant par ailleurs une tension de seuil et qui délivrent respectivement la synchronisation et le mot d'état QRS. Ce mot d'état QRS est envoyé à deux registres 56, 57 qui transforment les signaux série en signaux parallèles.

Le fonctionnement des circuits précédents s'explique de la façon suivante :

Soit

T0e la période du signal de référence à l'enregistrement,

T1e la période du signal ECG à l'enregistrement,

T01 la période du signal de référence à la lecture,

T11 la période du signal ECG à la lecture,

k le rapport de la vitesse de lecture à la vitesse d'enregistrement,

tc le temps de comptage,

n1 le résultat du comptage de T11,

n0 le résultat du comptage de T01,

On a :

$$tc = \frac{T01}{2 \cdot 128} = \frac{T0e}{2 \cdot k \cdot 128}$$

$$\frac{T11}{2} = n1 \, tc = \frac{T1e}{2 \, k}$$

$$\frac{T01}{2} = n0 \; tc = \frac{T0e}{2 \; k}$$

$$\frac{1}{n1} = \frac{2 \; k \; tc}{T1e}$$

$$\frac{1}{n0} = \frac{2 \; k \; tc}{T0e}$$

$$\frac{1}{n1} - \frac{1}{n0} = \frac{2 \; k \; tc}{T1e} - \frac{2 \; k \; tc}{T0e} = 2 \; k \; tc \left( \frac{1}{T1e} - \frac{1}{T0e} \right)$$

---

$$\frac{1}{n1} - \frac{1}{n0} = 2 \; ktc \; (f1 - f0)$$

---

$$\frac{1}{n1} - \frac{1}{n0} = \frac{T0e}{128} \; (f1 - f0)$$

$T0e$ étant constant,

$$(f1 - f0 = f) \text{ est égal à } \frac{1}{n1} - \frac{1}{n0}$$

à un facteur constant près.

On a donc bien réalisé la démodulation souhaitée.

Les moyens de lecture 5 présentent en outre un microprocesseur 58 relié à un dispositif de commande 59 communément appelé "souris". Ce microprocesseur 58 reçoit des informations des moyens d'entraînement 19 et du multiplicateur 46. Il est relié aux moyens d'entraînement 19 de la cassette par une logique de commande 60.

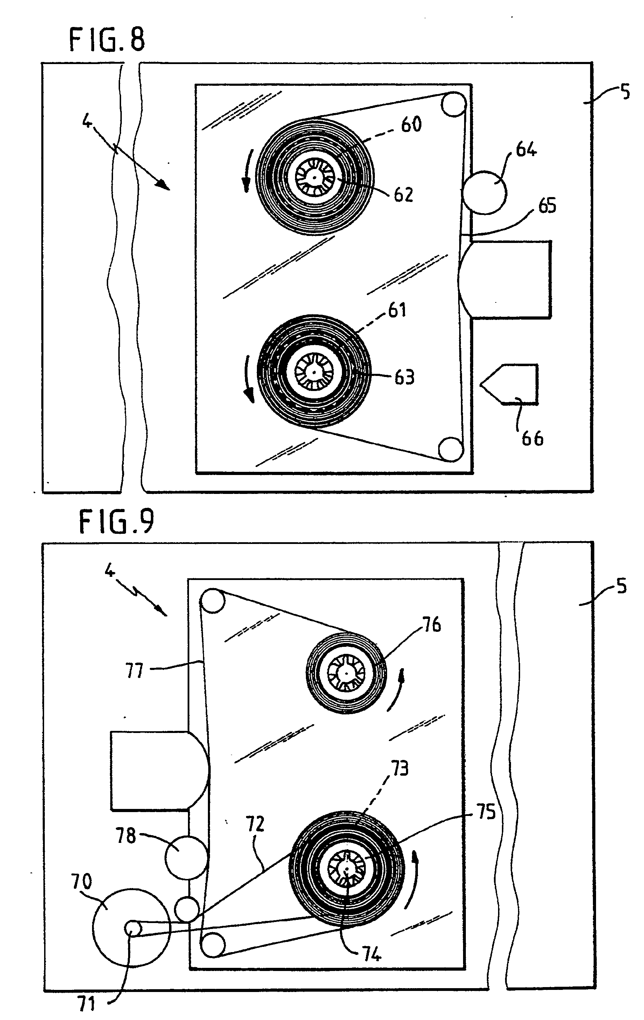

Comme on peut le voir sur la Figure 8, les moyens d'entraînement 19 de la cassette 4 lors de la lecture de la bande magnétique sont particulièrement simples, du fait que le système de démodulation s'affranchit de la vitesse de défilement de la bande magnétique.

Ils se composent de deux moteurs 60 et 61 coaxiaux aux bobines 62 et 63 de la cassette 4. En marche avant, le moteur 60 est actif et le moteur 61 sert de frein. En marche arrière, le moteur 61 est actif et le moteur 60 sert de frein. En position STOP, les moteurs 60 et 61 maintiennent la tension de la bande. Les moyens d'entraînement 19 présentent en outre un capteur optoélectronique 64 qui capte la vitesse linéaire de la bande magnétique 65 et régule la vitesse du moteur, ainsi qu'un capteur de fin de bande 66.

Comme on peut le voir sur la figure 9, les moyens d'entraînement 14 de la cassette 4 lors de l'enregistrement sont également très simples pour les raisons déjà évoquées. Ils se composent d'un moteur 70 dont l'arbre de sortie 71 reçoit une simple courroie 72 engagée par ailleurs sur une poulie 73 solidaire d'un embout d'entraînement standard 74 pour une bobine également standard 75 de la cassette 4. Une deuxième poulie 76 est associée à un frein de tension de la bande magnétique 77.

Le dispositif possède en outre un capteur optoélectronique 78 qui permet de mesurer la vitesse linéaire de la bande magnétique 77, évitant également tout risque de blocage.

Le temps de lecture peut être réduit par rapport au temps réel dans un rapport qui varie de 60 à 1000. c'est-à-dire qu'une période d'ECG de 24 heures peut être lue en :

$$\frac{24 \cdot 60}{1000} = 1,44 \text{ minute}$$

Les moyens de lecture 5 étant reliés au micro-ordinateur 8, celui-ci traite les mots d'état QRS et on peut obtenir en quelques minutes les résultats de l'analyse, tels que : courbes, comptages d'événement, S-T, séquences pathologiques significatives. La visualisation in extenso des signaux enregistrés sur la cassette 4 n'est nécessaire que si l'on désire une analyse plus fine de l'ECG ou si le résultat de l'analyse est insuffisant pour établir un diagnostic.

En outre, l'invention offre la possibilité de lecture et d'analyse à vitesse variable non seulement en bobinage mais également en rembobinage, ce qui évite cette opération lors de la lecture de la cassette d'où un gain de temps d'environ deux minutes, contrairement aux dispositifs connus qui n'autorisent deux ou trois vitesses différentes qu'avec le rembobinage, et encore s'agit-il de vitesses fixes et non de vitesse variable. Bien sûr, ces dispositifs ne permettent pas la lecture et l'analyse dans les deux sens.

Dans la pratique courante, la vitesse de lecture est définie par le cabestan, alors que l'invention permet de le supprimer.

Le dispositif selon l'invention a, alors, les fonctions suivantes :
– bobinage,
– rembobinage,
– stop,
– accélération/décélération/stop.

On a décrit un exemple de réalisation dans lequel des informations relatives aux tracés, comme le mot d'état QRS, sont obtenues en temps réel et sont transférées périodiquement sur une piste de la bande magnétique, alors que les tracés eux-mêmes sont transférés sur d'autres pistes. Dans le cadre de l'invention, on peut, lors de chaque transfert, conserver ces informations dans la mémoire solide et les lire à la fin d'une période de surveillance. Ainsi on n'a pas besoin de lire systématiquement la bande magnétique pour avoir connaissance des résultats, ce qui permet de gagner beaucoup de temps.

On a également décrit un exemple de réalisation dans lequel une bande magnétique sert de mémoire de masse. Cette solution offre une grande facilité d'exploitation car des vitesses très différentes peuvent être sélectionnées de façon continue. Mais on peut également utiliser comme mémoire de masse tout autre support d'enregistrement, par exemple des disques durs.

Enfin, on a décrit un dispositif faisant appel à une modulation de fréquence, mais on peut également, dans le cadre de l'invention, utiliser une modulation d'amplitude telle que la "période" du signal à la sortie soit proportionnelle à l'amplitude du signal à l'entrée et compter cette "période" au moyen de la fréquence de référence, ou d'un multiple de cette fréquence, suivant le procédé décrit précédemment.

La modulation d'amplitude permet ainsi de contrôler la vitesse.

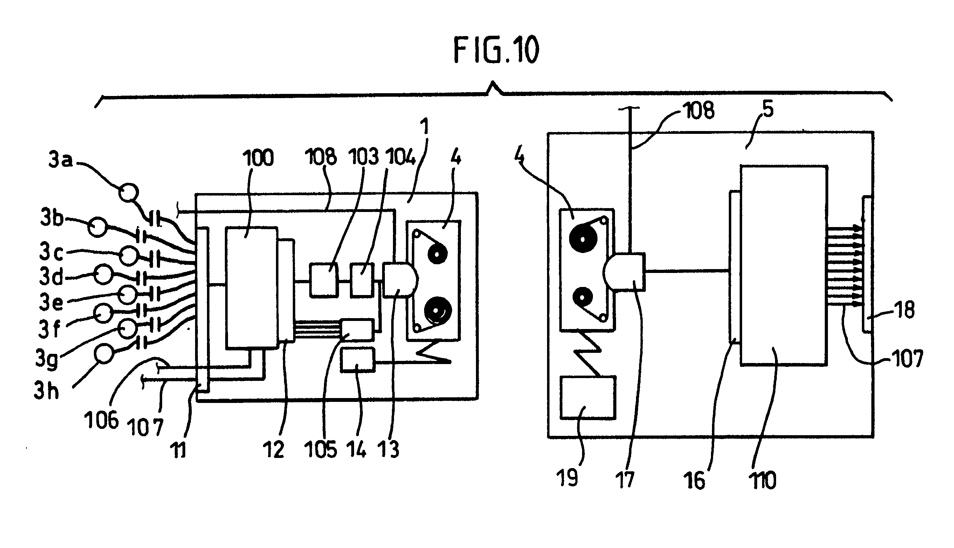

En se reportant maintenant aux figures 10 à 13, on voit un mode de réalisation de l'invention prévoyant le multiplexage des signaux, les éléments communs à ce mode de réalisation et à celui décrit précédemment portant les mêmes références.

Sur la figure 10, on voit le schéma général d'un appareil à huit voies, pour huit capteurs (ou "électrodes") 3a à 3h. Les conducteurs sont raccordés à une entrée 100 d'un multiplexeur 101 dont la sortie 102 est raccordée à un modulateur de fréquence 103 des signaux multiplexés, modulateur qui précède un diviseur de fréquence 104 (division par 128) dont les signaux de sortie sont envoyés d'une part à la tête d'écriture 13 et d'autre part à un décodeur d'adresses 105. Le modulateur 103 est un oscillateur contrôlé en fréquence.

Le multiplexeur 100 reçoit aussi un conducteur d'entrée 106 pour une tension calibrée et un conducteur d'entrée 107 pour le zéro électrique des capteurs 3.

La tension calibrée, de valeur connue, doit être supérieure à la tension la plus élevée susceptible d'être présente à l'entrée 11, par exemple 1,25 fois la tension maximale possible, afin de la reconnaître lors de la démodulation, soit par le niveau de tension décodé, soit par le comptage de la période, en numérique.

Cette tension calibrée a deux rôles :
– corriger le gain de l'ensemble des entrées, dû à la dérive des modulateurs et amplificateurs de l'appareil,
– assurer la synchronisation et lareconnaissance des voies lors de la lecture.

En effet, la tension calibrée et le zéro électrique permettent, lors de la lecture, de réaliser une compensation automatique de gain et de zéro, ce qui rend "transparentes", neutres, toutes les dérives de composants ou de vitesse, tant à l'enregistrement qu'à la lecture.

Il s'agit bien d'une compensation complémentaire à celles déjà décrites, celles-ci ne prenant pas en compte les éventuelles dérives des composants.

La tête d'écriture 13 reçoit une liaison 108 pour un signal de référence de fréquence.

On sait que la modulation de fréquence en multiplexage est rarement utilisée en raison de multiples difficultés techniques :
– synchronisation des voies lors de la lecture,
– difficultés de démodulation,
– variations de vitesse tant à l'enregistrement qu'à la lecture,
– temps de stabilisation important du modulateur 103 (1/2 période environ).

Selon l'invention, ces problèmes sont résolus du fait que le processus d'enregistrement, de stockage et de restitution des signaux n'est plus astreint à la régularité mécanique de la vitesse d'entraînement du support d'enregistrement.

De plus, si l'on souhaite simplement une double alternance de voies, comme on vient de le décrire, il faut disposer d'un modulateur dont le temps de stabilisation est compatible avec la précision recherchée. Si l'on recherche un nombre de points optimal, il faut plusieurs impulsions par voie multiplexée, ce qui a pour conséquence de diminuer la fréquence d'échantillonnage et, donc, la bande passante. Il est alors préférable d'adopter un autre type de modulation dit "modulation par densité d'impulsions" car ici la valeur du signal d'entrée est proportionnelle au temps qui sépare deux impulsions.

Ce problème est résolu selon l'invention et au moyen du diviseur de fréquence 104.

Le décodeur d'adresse étant situé à la sortie du diviseur de fréquence 104, il faut 128 périodes d'horloge du modulateur 103 pour chacune des voies d'entrée.

L'erreur du modulateur 103 est donc divisée par 128, étant bien entendu que l'on peut adopter un plus grand diviseur pour obtenir une précision plus importante.

Les signaux multiplexés en fréquence sont toujours carrés et l'erreur de phase est minorée lors de la lecture.

En ce qui concerne la synchronisation des voies, il faut préciser que la modulation par multiplexage doit nécessairement être combinée avec le procédé de démultiplexage conforme à l'invention : comptage de la période de base du signal modulé en fréquence et correction des instabilités ("jitter") ainsi que des variations de vitesses d'enregistrement et de lecture par la fréquence de référence, le tout, bien entendu, en s'affranchissant des problèmes de vitesses et notamment par l'utilisation de cinématiques simplifiées.

Le démultiplexage est, en soi, bien connu et ne sera donc pas décrit en détail. Le schéma de la partie lecture de l'appareil est identique pour chaque voie à celui que l'on a déjà commenté et sur la figure 10, on a représenté un simple bloc électronique 110, sachant que celui-ci doit nécessairement comprendre un démultiplexeur.

Le nombre de voies dépend, pour chaque voie, de la bande passante désirée, de la vitesse de la bande magnétique lors de l'enregistrement, de la durée de l'enregistrement.

La détection du synchronisme des voies est effectuée par un comparateur de tension.

La sortie 107 du zéro peut corriger le zéro des voies car elle correspond aux différentes dérives des composants :
– erreur du modulateur 103 lors de l'enregistrement,
– erreur du modulateur 103 lors de la démodulation,
– dérive des amplificateurs d'enregistrement et de lecture.

La tension de zéro est sommée avant amplification, au même titre que la correction des instabilités ("jitter").

La tension calibrée constitue une référence pour la conversion 1/T et contrôle ainsi le gain de la chaîne de composants.

Bien entendu, cette tension est comparée à une référence de tension interne avant de contrôler le gain de l'ensemble.

On obtient, de la sorte, un gain automatique donc une très bonne stabilité de l'ensemble.

## Revendications

1- Procédé pour l'enregistrement et la lecture de signaux, notamment d'électrocardiogrammes, caractérisé en ce que l'on enregistre en continu la totalité des signaux émis, dans au moins une mémoire solide, que l' on transfère périodiquement lesdits signaux sur un support d'enregistrement après leur avoir fait subir une modulation, que l'on enregistre sur ledit support d'enregistrement une fréquence de référence, ladite fréquence servant à la démodulation des signaux, que l'on traite les signaux en temps réel lors de l'enregistrement, que l'on stocke les informations obtenues dans une partie de la mémoire solide qui n'est pas affectée lors des transferts périodiques, afin de pouvoir lire ces informations sans faire appel au support d'enregistrement comportant un tracé complet des signaux captés.

2- Procédé selon la revendication 1, caractérisé en ce quele support d'enregistrement est une bande magnétique etlors de la lecture, on compte la période de la fréquence de référence et la période de la fréquence des signaux en utilisant comme fréquence d'horloge une fréquence multiple de la fréquence de référence et on fait la différence de l'inverse des périodes obtenues de façon à restituer les signaux enregistrés en s'affranchissant de la vitesse et des variations de vitesse de la bande magnétique, aussi bien à l'enregistrement qu'à la lecture.

3- Procéde selon la revendication 1, caractérisé en ce que l'on transfère périodiquement des signaux de la mémoire solide au support d'enregistrement à une vitesse supérieure à la vitesse d'écriture dans la mémoire solide.

4- Procédé selon la revendication 1, caractérisé en ce que, lors de l'enregistrement, on choisit comme vitesse de déroulement de la bande magnétique une vitesse standard telle que neuf centimètres par seconde.

5- Procédé selon la revendication 1, caractérisé en ce que l'on enregistre les signaux multiplexés en modulation de fréquence sur un support d'enregistrement, puis on les démultiplexe afin de les reconnaître.

6- Procédé selon la revendication 1, caractérisé en ce que l'on amplifie les signaux et on compense le gain qui en résulte ainsi que des variations de paramètres affectant l'enregistrement des signaux tels que la vitesse d'enregistrement.

7- Procédé selon la revendication 1, caractérisé en ce que l'on synchronise les signaux à enregistrer et on les repère par rapport à un signal de référence.

8- Dispositif pour l'enregistrement et la lecture de signaux, notamment d'électrocardiogrammes, du type comprenant un support d'enregistrement, des moyens d'entraînement du support d'enregistrement, une tête d'écriture et une tête de lecture reliées respectivement à un bloc électronique d'écriture et à un bloc électronique de lecture, caractérisé en ce qu'il présente une liaison entre les blocs électroniques et les têtes, liaison destinée à un signal de référence de vitesse.

9- Dispositif selon la revendication 8, comprenant une cassette à bande magnétique, des moyens d'entraînement de la bande magnétique, une tête magnétique d'enregistrement et une tête magnétique de lecture reliées respectivement à un bloc électronique d'enregistrement et à un bloc électronique de lecture, caractérisé en ce qu'il présente une liaison entre les blocs électroniques et les têtes magnétiques, liaison déstinée à un signal de référence de vitesse.

10- Dispositif selon la revendication 8, du type comprenant des moyens d'enregistrement pouvant recevoir une cassette et reliés à des capteurs, caractérisé en ce qu'il possède une liaison reliant le bloc électronique d'enregistrement et la tête magnétique d'enregistrement, liaison déstinée à un signal de référence de vitesse.

11- Dispositif selon la revendication 8, du type comprenant des moyens de lecture pouvant recevoir une cassette et reliés à un ordinateur et/ou à une imprimante, caractérisé en ce qu'il possède une liaison reliant le bloc électronique de lecture et la tête magnétique de lecture, liaison destinée un signal de référence de vitesse.

12- Dispositif selon la revendication 8, caractérisé en ce que le bloc électronique d'enregistrement comprend un oscillateur à quartz pour générer une fréquence servant de signal de référence de vitesse.

13- Dispositif selon la revendication 8, caractérisé en ce que le dispositif comprend un oscillateur contrôlé en tension dont la fréquence centrale est égale à la fréquence de l'oscillateur à quartz.

14- Dispositif selon la revendication 8, caractérisé en ce que le bloc électronique de lecture comprend un circuit multiplicateur destiné à multiplier le signal de référence de vitesse par une constante.

15- Dispositif selon la revendication 8, caractérisé en ce que le circuit multiplicateur est un circuit à verrouillage de phase.

**16-** Dispositif selon la revendication 8, <u>caractérisé en ce que</u> le bloc électronique de lecture comprend des circuits compteurs devant être associés à des moyens d'horloge de comptage, ces moyens étant constitués par la fréquence multiple de la fréquence du signal de référence, délivrée par le circuit multiplicateur.

**17-** Dispositif selon la revendication 8, <u>caractérisé en ce que</u> le bloc électronique d'enregistrement présente une mémoire solide et des décodeurs d'adresse dont la fréquence de travail est égale ou sous-multiple de la fréquence de référence lors de l'écriture et est égale ou multiple de la fréquence de référence lors de la lecture.

**18-** Dispositif selon la revendication 8, <u>caractérisé en ce que</u> le bloc électronique d'enregistrement comporte au moins une voie destinée à recevoir un signal sous forme analogique, une voie destinée à recevoir un signal de référence calibré et une voie de mise à zéro.

**19-** Dispositif selon la revendication 8, <u>caractérisé en ce que</u> le bloc électronique d'enregistrement comporte un multiplexeur associé à des moyens de modulation en fréquence et à un organe de décodage d'adresses.

**20-** Dispositif selon la revendication 8, <u>caractérisé en ce que</u> le bloc électronique de lecture comporte un démultiplexeur associé à un échantillonneur.

**21-** Dispositif selon la revendication 8, <u>caractérisé en ce que</u> les moyens d'entraînement comprennent un moteur qui est relié soit directement, soit au moyen d'une simple courroie, à une poulie solidaire d'un embout d'entraînement devant être en prise avec une bobine d'une cassette.

**22-** Dispositif selon la revendication 8, <u>caractérisé en ce que</u> les moyens d'entraînement comprennent deux moteurs qui sont coaxiaux à deux bobines d'une cassette.

**23-** Dispositif selon la revendication 8, <u>caractérisé en ce que</u> les moyens d'entraînement sont associés à un ensemble optoélectronique de contrôle et de régulation de la vitesse de la bande magnétique d'une cassette.

FIG.1

FIG.2

FIG.3

FIG.5

EP 0 461 009 A1

FIG.4

EP 0 461 009 A1

FIG.6

EP 0 461 009 A1

FIG.7

EP 0 461 009 A1

FIG.8

FIG.9

FIG.10

Office européen
des brevets

**RAPPORT DE RECHERCHE EUROPEENNE**

Numero de la demande

EP    91 40 1414
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,X | EP-A-3797 (SIEMENS AKTIENGESELLSCHAFT)<br>* figure 1 *<br>* page 4, ligne 17 – page 7, ligne 30 *<br>* page 8, ligne 22 – page 9, ligne 17 * | 8 | A61B5/0436 |
| A | | 1, 6, 9-11 | |
| | --- | | |
| D,X | US-A-4696306 (SHIOZAKI)<br>* figures *<br>* colonne 2, ligne 50 – colonne 4, ligne 13 * | 8 | |
| A | | 1, 9, 10 | |
| | --- | | |
| D,X | US-A-4883065 (KELEN)<br>* figures 1-3 *<br>* colonne 4, ligne 15 – colonne 6, ligne 18 * | 8 | |
| A | | 1, 6, 9-12, 18 | |
| | --- | | |
| X | US-A-3650263 (KOWALSKI ET AL.)<br>* figures 1, 2, 4 *<br>* colonne 3, ligne 7 – colonne 7, ligne 53 *<br>* colonne 10, ligne 4 – colonne 11, ligne 18 * | 8 | |
| A | | 1, 6, 9, 10, 13 | **DOMAINES TECHNIQUES RECHERCHES (Int. Cl.5)**<br><br>A61B<br>G11B |
| | --- | | |
| D,A | GB-A-2034046 (BENNISH)<br>* figure 1 *<br>* page 1, ligne 93 – page 2, ligne 63 *<br>* page 5, lignes 26 – 49 * | 1, 8-10 | |
| | --- | | |
| A | FR-A-2387456 (ETAT FRANCAIS, DELEGUE GENERAL POUR L'ARMEMENT)<br>* figure 1 *<br>* page 2, ligne 33 – page 7, ligne 27 * | 1, 5, 18, 19 | |
| | --- | | |
| | -/-- | | |

Le présent rapport a été établi pour toutes les revendications

| Lieu de la recherche | Date d'achèvement de la recherche | Examinateur |
|---|---|---|
| LA HAYE | 10 SEPTEMBRE 1991 | CHEN A.H. |

**CATEGORIE DES DOCUMENTS CITES**

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
...........................................................................
& : membre de la même famille, document correspondant

EPO FORM 1503 03.82 (P0402)

Office européen
des brevets

**RAPPORT DE RECHERCHE EUROPEENNE**

Numero de la demande

EP    91 40 1414
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | MEDICAL AND BIOLOGICAL ENGINEERING AND COMPUTING. vol. 23, no. 6, novembre 1985, STEVENAGE GB pages 607 - 609; BUNDY et al.: "ECG tape-speed error compensator." * le document en entier * | 1, 14, 15 | |
| A | US-A-3675876 (FREDERICK) * figure 1 * * colonne 2, ligne 17 - colonne 3, ligne 33 * | 21 | |
| A | US-A-3764087 (PAANANEN) * figure 1 * * colonne 4, ligne 16 - colonne 6, ligne 62 * | 22, 23 | |
| | | | **DOMAINES TECHNIQUES RECHERCHES (Int. Cl.5)** |

Le présent rapport a été établi pour toutes les revendications

| Lieu de la recherche | Date d'achèvement de la recherche | Examinateur |
|---|---|---|
| LA HAYE | 10 SEPTEMBRE 1991 | CHEN A.H. |